# EUROPEAN PATENT APPLICATION

(11) **EP 4 763 073 A1**
(43) Date of publication of application: **24.06.2026**
(21) Application number: 24220751.2
(22) Date of filing: 17.12.2024
(51) Int. Cl.: A61B 5/08, A61B 5/00, G16H 50/20

(54) **METHOD FOR IDENTIFYING A PATIENT'S RESPIRATORY, IN PARTICULAR COUGH ACTIVITY AMONG OTHER PATIENT S RESPIRATORY, IN PARTICULAR COUGH ACTIVITIES**

(71) Applicant: SIVA Health AG, 8003 Zürich (CH)
(72) Inventor: GISLER, Daniel Robert, 8003 Zürich (CH); ALGE, Mitja Stefan, 8003 Zürich (CH); DUSCHAU-WICKE, Alexander Herbert Benjamin, 8003 Zürich (CH)
(74) Representative: Maiwald GmbH

(57) **Abstract**

A method and a system for identifying an individual person's respiratory activity among a group of persons' respiratory activities, which allows an iterative verification of respiratory activity data sets of a person.

## Description

### Field of the Invention

The present invention relates to a method for identifying a patient's respiratory, in particular cough activity among other patient's respiratory, in particular cough activities and to teach a system and data base, respectively, for a more reliable identification of a patient and its corresponding respiratory, in particular cough activity and a system which allows carrying out the method, as well as a system for evaluating respiratory, in particular coughing activities, which method and system allow a more reliable evaluation of a respiratory, in particular coughing activity of a user or a patient.

### Background of the Invention

Coughing is an indication for a disease. Coughing may be acute or may be chronic and may also change overtime. The characteristic of coughing may be an indicative for a disease or an intensity of a disease. It may be valuable to evaluate the respiratory, in particular coughing activity of a patient in order to conclude a disease, so as to treat the disease properly.

For an untrained observer, a respiratory, in particular coughing activity may be random, and it may be difficult to have an objective measure which may serve for a proper diagnostic. Further, the observation period of a human observer is rather short, as the observation by a human observer lasts only a short time, e.g., only a couple of minutes. For this purpose, sensing and recording devices were used in the prior art, which however did not allow a proper evaluation of the sensed cough activities. For cough sensing and monitoring a system may be used which are capable of detecting a cough activity of a patient, e.g., audio data of a cough activity. However, it is not uncommon that the patient to be monitored during sensing and monitoring is not alone and accompanied by other persons, e.g., the spouse or family members or other patients, e.g., in hospital or when visiting a physician. Coughs detected via audio features can come from a person which is not the monitored patient (foreign cougher). It is generally difficult to assign a respiratory activity, in particular coughs to a person based on only the audio of the cough, even for expert human cough labelers.

Approaches in prior art are described in Jokić, Stefan, David Cleres, Frank Rassouli, Claudia Steurer-Stey, Milo A. Puhan, Martin Brutsche, Elgar Fleisch, und Filipe Barata, 2022, *«TripletCough: cougher identification and verification from contact-free smartphone-based audio recordings using metric learning»,* IEEE Journal of Biomedical and Health Informatics 26(6):2746-57, https://ieeexplore.ieee.org/stamp/stamp.jsp?tp=&arnumber=9720192; in Schroff, Florian, Dmitry Kalenichenko, und James Philbin. 2015, «Facenet: A unified embedding for face recognition and clustering», S. 815-23 , Proceedings of the IEEE conference on computer vision and pattern recognition, https://arxiv.org/pdf/1503.03832; and in Hermans, Alexander, Lucas Beyer, und Bastian Leibe, 2017, «In Defense of the Triplet Loss for Person Re-Identification», https://arxiv.org/pdf/1703.07737.

Therefore, there is a need for providing a method and a system for identifying a patient along a respiratory, in particular cough fingerprint of that patient and to determine reliable data for respiratory, in particular cough monitoring and evaluation.

### Summary of the Invention

The present invention provides a method and device for identifying an individual person's respiratory activity among a group of persons' respiratory activities according to the independent claims, whereas further embodiments are incorporated in the dependent claims.

### For the present invention the following definitions should apply:

A respiratory event is considered as an activity of the or in connection with a respiratory tract of a person, which may be a respiratory sound event or a respiratory motion/acceleration event or a combination thereof, which may include coughs, throat clearings, sneezes, wheezing, crackles and similar activities, which may result from an active action or a reflex action of said person.

A respiratory activity is considered as a respiratory event sequence including one or more respiratory events, based on an initial trigger stimulus.

Respiratory activity data are considered as an unspecific data stream of a recorded signal resulting from a respiratory activity.

A respiratory activity data set is considered as a specific data set including allocations, correlations or relations between a signal characteristic and parameters or features of a respiratory activity (labeled data).

A cough event is considered as a single cough pulse, or a sequence of cough pulses with a temporal difference between the cough pulses below a predetermined period. A cough or cough event may be considered as a part of a respiratory activity of a user or a patient, which activity is a reflex to cough or clear the throat upon a stimulus inside or outside the patient's respiratory tract. Usually, a cough event occurs repeatedly a couple of times with different periods in between where no cough event takes place.

A cough activity is considered as a cough event sequence including one or more cough events, based on an initial trigger stimulus.

Cough data are considered as an unspecific data stream of a recorded signal resulting from a cough activity.

A cough data set is considered as a specific data set including allocations, correlations or relations between a signal characteristic and parameters or features of a cough activity (labeled data).

An audio score is considered as a score, which may also be a scalar or one-dimensional score, being indicative (as scalar value) how likely audio data of a cough or respiratory activity origin from an individual person or a predetermined person.

An acceleration score is considered as a score, which may also be a scalar or one-dimensional score, being indicative (as scalar value) how likely acceleration data of a cough or respiratory activity origin from an individual person or a predetermined person.

An embedding score is considered as a score, which may also be a scalar or one-dimensional score, being indicative (as scalar value) how likely a cough or respiratory activity origins from an individual person or a predetermined person. How likely a cough or respiratory activity origins from an individual person or a predetermined person may be expressed by a similarity of the cough or respiratory activities or cough or respiratory activity data between the received ones and the individual person ones and the predetermined person ones, respectively.

An individual person is considered as the person of interest, which may be a patient, for which the respiratory activities are to be allocated and evaluated and for which person an identification of diseases may be carried out.

A predetermined person is considered as a further person beside the individual person, to which predetermined person also respiratory activities may be allocated, however not primarily for evaluating the same, but for sake of not allocating its respiratory activity to the individual person. In other words, when allocating the respiratory activity reliably to the predetermined person, an allocation to the individual person usually is excluded. It is however not excluded that also for the predetermined person allocated respiratory activities are evaluated, but then rather as a second individual person beside the (first) individual person.

A group of persons is considered as a group of persons including mandatorily the individual person and optionally the predetermined person. The group of persons is not limited to a particular number of persons. The group of persons may include one or more individual persons in the meaning of the above definition of the individual person and may also include one or more predetermined persons in the meaning of the above definition of predetermined persons.

### The subject matter of the invention is defined as follows:

According to an embodiment, there is provided a method for identifying an individual person's respiratory activity among a group of persons' respiratory activities, the method comprises iteratively providing an individual person's verified respiratory activity data set based on pre-collected respiratory activity data of said individual person's respiratory activities; receiving respiratory activity data of a respiratory activity of at least one person out of a group of persons recorded by a respiratory activity recording device; determining from the received respiratory activity data a set of respiratory data scores; identifying the received respiratory activity data of a respiratory activity to be an event out of an event group, the event group consisting of an event of said individual person's respiratory activity and an event of a respiratory activity of a person different from the individual person, wherein identifying the received respiratory activity data of a respiratory activity is based on the individual person's verified respiratory activity data set and a given set of respiratory activity identification decision rules; confirming whether the received respiratory activity data origin from the individual person based on the determined set of respiratory data scores and a set of individual person confirmation decision rules; upon confirming: updating the individual person's verified respiratory activity data set based on the received and confirmed respiratory activity data; providing the respiratory activity data of a respiratory activity identified as an event of said individual person's respiratory activity for further evaluation of said individual person's respiratory activity.

Thus, the method provides a possibility to identify a respiratory activity and to allocate the same to a patient or in general to an individual person based on verified respiratory activity data coming from that patient or individual person, respectively. The verified data can be considered as verified as they are pre-collected from said patient or individual person and at the same time it can be excluded that these data stem from another person. This can be achieved by recording patient's or individual person's data in an environment which is not impacted by other persons respiratory activity data. Identification can be carried out by comparing characteristics and respiratory activity parameters upon application of respiratory activity identification decision rules, which are provided for identification. The respiratory activity identification decision rules may be stored and defined beforehand. The respiratory activity identification decision rules may be applicable to a large number of persons. After confirming the allocation with a high certainty to the patient or individual person based on respiratory data scores, the patient's or individual person's verified respiratory activity data may be updated to enlarge the data basis for the individual person's verified respiratory activity data. Updating may be carried out upon determination of a certainty requirement, e.g. only in case the certainty is determined to achieve a particular level of e.g. 99%.

Identifying the received respiratory activity data of a respiratory activity to be a respiratory activity related to a respiratory activity of an individual person or a respiratory activity of a person different from the individual person based on the individual person's verified respiratory activity data set and a given set of respiratory activity identification decision rules serves for allocating the received respiratory activity data to an individual person. After identifying the source or origin of the received respiratory activity, the respective respiratory activity data may be provided for evaluation and further diagnosis. The identification may be considered as creating a best guess to produce data for further evaluation.

If it is intended to use the source or origin of identified respiratory activity data for updating the verified respiratory activity (e.g. cough activity) data stemming from a particular source, it may be desirable to apply a confirmation. For such confirmation it may be desirable to allocate the received respiratory activity data to an individual person only in cases where it is very likely that the received respiratory activity data actually relate to said individual person. For this purpose, the set of respiratory activity confirmation decision rules may be designed to provide a large certainty of the allocation to said individual person, e.g. a certainty of 99%. With this respect it may be acceptable to erroneously allocate a respiratory activity coming from the individual person to be a respiratory activity from a person different than the individual person, as long as the certainty is high that respiratory activity allocated to the individual person actually stem from said individual person. Confirmation of a respiratory activity (e.g., coughs) to be added to the verified data may only be carried out if a respective respiratory activity (e.g., coughs) is safely allocated to the monitored person so that upon confirmation the data can be or are added to the verified dataset.

According to an embodiment, the respiratory data scores include an audio score, wherein the audio score is representative for a physical distance between a person and the respiratory activity recording device.

Thus, the identification and confirmation can be carried out on a score, which is based on an acoustic signal, which can be recorded by the wearable device. As a simple score a level and damping of a signal can be evaluated as indicative for a distance between the recording device and the patient or individual person. However, also more advanced analysis can be made based on audio signals. Acoustic audio data are easily processable and can be undertaken also advanced analysis and data processing in the time domain and the frequency domain, e.g. by applying Fourier analysis, convolutions and other analysis. In particular, the already verified respiratory activity data of the patient or individual person can be compared with the recorded data, e.g. by convoluting or cross correlating the signals. The results thereof can be used for identifying and confirming the origin of the respiratory activity data.

According to an embodiment the respiratory data scores include an acceleration score, wherein the acceleration score is representative for an acceleration of a person's anatomy during a respiratory activity.

Thus, the identification and the confirmation can be carried out on a score, which is based on a motion, acceleration and/or jerk signal, which can be recorded by the wearable device. For this purpose, the recording device may be equipped with a gyroscope or an accelerometer. Acceleration can be detected and measured in all three spatial directions and along all three rotational directions. As an alternative also motion can be detected, wherein detecting a motion over time allows determination of an acceleration. Likewise, an acceleration may be detected over time which may allow determination of a jerk. It should be noted that as an alternative or in addition to the acceleration score also a motion score or a jerk score may be determined and may be used for confirming the origin of respiratory activity data. In particular the already verified respiratory activity data of the patient or individual person can be compared with the recorded data, e.g. by convoluting or cross correlating the signals for determining the respective scores. The results thereof can be used for identifying and confirming the origin of the respiratory activity data.

According to an embodiment, confirming the received respiratory activity data of a respiratory activity is based on the individual person's verified respiratory activity data set, a given set of respiratory activity confirmation decision rules and an individual person's embedding score, wherein the embedding score is representative for a confidence level of the identification of a respiratory activity to be an event of said individual person's respiratory activity.

Thus, the certainty of the confirmation can be improved, so that the verified data are only updated if it is clear that the respiratory data origin from the patient or the individual person, respectively. If in addition to a generally high data score the embedding score of the patient or individual person is e.g. high, the certainty is high that the recorded respiratory activity data origin from a patient or individual person. The respiratory activity confirmation decision rules may be generic, but also may be related to a particular person, e.g., a patient or a predetermined person beside the patient. With this respect the respiratory activity confirmation decision rules may be calibrated to a particular person. The respiratory activity confirmation decision rules may also include rules considering a particular situation, e.g., if a patient is asked upon start of application, whether there are other individuals present, e.g., at night. If so, the respiratory activity confirmation decision rules may be adapted to consider this. If not, the respiratory activity confirmation decision rules may be adapted accordingly, e.g. in a way that it is very likely that the respiratory activity stems from said patient. The respiratory activity confirmation decision rules may be adapted according to an individual person/patient and may also be adapted to a further person.

According to an embodiment, confirming the received respiratory activity data of a respiratory activity is based on the individual person's verified respiratory activity data set, a given set of respiratory activity confirmation decision rules, an individual person's embedding score and a predetermined person's embedding score, wherein the respective embedding score is representative for a confidence level of the identification of a respiratory activity to be an event of said predetermined person's respiratory activity.

Thus, the certainty of the confirmation can be further improved, so that the verified data are only updated if the respiratory data origin from the patient or the individual person, and not from another predetermined person or a foreign person. If in addition to a generally high data score the embedding score of the other/predetermined person is high, it may be concluded that the recorded respiratory activity data do not origin from the patient/individual person, so that the certainty is low. If e.g. the embedding score of the patient or individual person is high, and if the embedding score of the predetermined person is also high, it may be concluded that the likelihood that the recorded respiratory activity data origins from the patient/individual person is similar to the likelihood that the respiratory activity data origins from the other/predetermined person. This means that the certainty to allocate the recorded respiratory activity data to the patient/individual person is low. Thus, consideration of the embedding score also of a another or predetermined person allows a more certain allocation of the recorded respiratory activity data. The same applies likewise for the respiratory activity confirmation decision rules as outlined above. With this respect the respiratory activity confirmation decision rules may be calibrated to a particular person. The respiratory activity confirmation decision rules may also include rules considering a particular situation, e.g., if a patient is asked upon start of application, whether there are other individuals present, e.g., at night. If so, the respiratory activity confirmation decision rules may be adapted to consider this. The respiratory activity confirmation decision rules may be adapted according to an individual person/patient and may also be adapted to a further person.

According to an embodiment the individual person's embedding score is determined based on a difference between received respiratory activity data and individual person's verified respiratory activity data set.

Thus, the individual person's embedding score can be provided as an indicative of the difference between received respiratory activity data and individual person's verified respiratory activity data set, which difference can be determined, e.g., by using a machine learning model which generates a multi-dimensional vector space from which the distances representing the above mentioned differences can be determined between the received respiratory activity data and the individual person's verified respiratory activity data set. The machine learning model may learn from audio data and/or acceleration data and further parameters and a respective labeling of the training data. The learned model then may allocate received e.g. audio data and/or acceleration data to a position in an n-dimensional space. Usually, audio and acceleration data from the same person are located in the n-dimensional space close to each other. The embedding score as a scalar value may e.g. be determined by computing an average distance between a number of respiratory activities/coughs of the received respiratory activity data and the verified respiratory activity data set. The average distance then may form a basis for a decision rule, which may distinguish between an embedding score above and below a threshold, respectively, which means that the averaged distance is above and below a level, respectively, which level is indicative whether a respiratory activity is allocated to a particular or individual person or not. The machine learned model may be trained by a Triplet Learning Process.

According to an embodiment predetermined person's embedding score is determined based on a difference between received respiratory activity data and predetermined person's verified respiratory activity data set.

Thus, the predetermined person's embedding score can be provided as an indicative of the difference between received respiratory activity data and predetermined person's verified respiratory activity data set, which difference can be determined, e.g., by using a machine learning model which generates a multi-dimensional vector space from which the distances representing the above mentioned differences can be determined between the received respiratory activity data and the predetermined person's verified respiratory activity data set.

According to an embodiment at least one of the individual person's embedding score and the predetermined person's embedding score is determined further based on at least one of a audio score and an acceleration score of the individual person and the predetermined person, respectively.

Thus, the audio score and/or the acceleration score as a representation of a person's respiratory activity like a cough activity can be used to further express the characteristic of a respiratory activity, which is expressed as an embedding score. It should be noted that the embedding score of a person can be based not only on the difference between received respiratory activity data and a verified respiratory activity data set of the respective person, but also on one or both of the audio score and the acceleration score but is not limited to these scores. It should be noted that also other parameters, like personal anamneses parameters may form a basis for the respective embedding score of a person, be it the patient/individual person or the other/predetermined person.

According to an embodiment the respiratory activity identification decision rules and or confirmation decision rules are represented by at least one of a look-up table, an algorithm and a model representing a relation between the respiratory data scores and the events out of the event group.

Thus, the respiratory activity identification and/or confirmation decision rules may be related to a relation of respiratory data scores and the events out of the event group. It should be noted that the respiratory activity identification and/or confirmation decision rules may be adapted during the process with the time and may underly a machine learning process, in particular including a feedback loop. It should be noted that the respiratory activity identification and/or confirmation decision rules may be general not personalized rules or may be rules related to a person, in particular to the patient/individual person and the other/predetermined person. Likewise, the audio scores and the acceleration scores may be generally related or related to a person.

According to an embodiment, the method further comprises providing for at least one predetermined person out of the group of persons a predetermined person's verified respiratory activity data set based on pre-collected respiratory activity data of said predetermined person's respiratory activities , wherein the event group further comprises the event of respiratory activity of the at least one predetermined person out of the group of persons, wherein identifying the respiratory activity data of a respiratory activity to be an event of the event group, is based on a set of respiratory activity identification decision rules, the individual person's verified respiratory activity data set, the predetermined person's verified respiratory activity data set and the set of respiratory data scores.

Thus, for identification and confirmation also more detailed information/data of not only the patient/individual person can be considered but also of another/predetermined person. The certainty of the identification and confirmation can be further improved. If it is determined that the recorded respiratory activity data correlate to the verified data of the other/predetermined person, then it can be excluded that the recorded respiratory activity data do not origin from the patient/individual person. If e.g. the recorded respiratory activity data correlate to the verified data of the patient or individual person and also the recorded respiratory activity data correlate to the verified data of the predetermined person the recorded respiratory activity data may be used for evaluating the respiratory activity of the patient/individual person but the recorded respiratory activity data will not be used for updating the verified data of the patient/individual person, as the certainty is not high enough, as there is a residual likelihood that the recorded respiratory activity data stem from the other/predetermined person. Thus, consideration of a verified respiratory activity data set of a another or predetermined person allows a more certain update of the verified respiratory activity data set of the patient/individual person.

According to an embodiment upon failed confirming or a bypassed confirming: the received respiratory activity data are provided for verification by an expert; and upon verification by an expert: updating the individual person's verified respiratory activity data set or predetermined person's verified respiratory activity data set based on the received and verified respiratory activity data.

Thus, the updating of the respective verified respiratory activity data set can be supported by providing the recorded respiratory activity data to an expert. It should be noted that the interface for expert confirmation outbound is the provision of the recorded respiratory activity data and the interface inbound is the reception of a confirmation or denial of confirmation. Recorded respiratory activity data may be provided to the expert for confirmation upon failed identification and confirmation by the method, or if the recorded respiratory activity data are found to be somewhere between certain confirmation/update of verified data and certain denial of confirmation.

According to an embodiment upon failed confirming or a bypassed confirming: the received respiratory activity data are provided for verification by said individual person; and upon verification: updating the individual person's verified respiratory activity data set based on the received and verified respiratory activity data.

Thus, the updating of the verified respiratory activity data set of the patient/individual person can be self-supported by providing the recorded respiratory activity data to the patient/individual person itself. It should be noted that the interface for self-confirmation outbound is the provision of the recorded respiratory activity data and the interface inbound is the reception of a self-confirmation by a patient/individual person. Recorded respiratory activity data may be provided to the patient/individual person for self-confirmation for pre-collecting verified respiratory activity data or upfront before the recorded respiratory activity data are provided for identification and confirmation. Identification and confirmation in this case may be not necessary, as the concerned patient/individual person directly confirms its own respiratory activity.

According to an embodiment upon failed confirming or a bypassed confirming: the received respiratory activity data are provided for verification by said predetermined person; and upon verification: updating the predetermined person's verified respiratory activity data set based on the received and verified respiratory activity data.

Thus, the updating of the verified respiratory activity data set of the other/predetermined person can be self-supported by providing the recorded respiratory activity data to the other/predetermined person itself. It should be noted that the interface for self-confirmation outbound is the provision of the recorded respiratory activity data and the interface inbound is the reception of a self-confirmation by the other/predetermined person. Recorded respiratory activity data may be provided to the other/predetermined person for self-confirmation for pre-collecting verified respiratory activity data or upfront before the recorded respiratory activity data are provided for identification and confirmation. Identification and confirmation in this case may be not necessary, as the concerned other/predetermined person directly confirms its own respiratory activity.

According to an embodiment for determination of the embedding score the respiratory activity data of a number of respiratory activities of the individual person's verified respiratory activity data sets are clustered as a verified cluster in an in an n-dimensional embedding model space, wherein the embedding score is determined as a distance between the received respiratory activity data of a respiratory activity and the verified cluster in the embedding model space. The distance may be a complex distance function.

Thus, the respective embedding score may be determined more accurately so that the confirmation of recorded respiratory activity data can be made more reliable.

According to an embodiment, upon receiving of respiratory activity data of a respiratory activity and identification of said respiratory activity data of a respiratory activity to be an event of individual person's respiratory activity, the number of respiratory activities of the individual person's verified respiratory activity data set is supplemented based on the received respiratory activity data, and the supplemented individual person's verified respiratory activity data set is re-clustered as an updated verified cluster, in particular in an in an n-dimensional embedding model space.

Thus, the amount of verified respiratory activity data sets can be iteratively enlarged and extended to further improve the identification and confirmation process for further recorded respiratory activity data.

According to an embodiment determining the embedding score is based on the individual person's verified respiratory activity data set updated based on recently received respiratory activity data related to the individual person's respiratory activity.

Thus, the determination of the respective personal embedding score can be improved under consideration of successively identified and confirmed respiratory activity data related to the patient/individual person.

According to an embodiment a system is provided for identifying an individual person's respiratory activity among other person's respiratory activities, the system comprises: a wearable respiratory activity recording device being adapted for sensing and recording respiratory activity data of a respiratory activity of at least one person out of a group of persons including an individual person; a processing unit having access to an individual person's verified respiratory activity data set based on pre-collected respiratory activity data of said individual person's respiratory activities; wherein the processing unit comprises: a respiratory data score determination entity which is adapted for determining from the received respiratory activity data, a set of respiratory data scores; a respiratory event identification entity which is adapted for identifying the received respiratory activity data of a respiratory activity to be an event out of a group, the group comprising the event of said individual person's respiratory activity and the event of a respiratory activity of a person different from the individual person, wherein identifying the received respiratory activity data of a respiratory activity is based on a given set of respiratory activity identification decision rules; a confirmation entity, which is adapted for confirming whether the received respiratory activity data origin from the individual person based on the set of respiratory data scores and a set of individual person confirmation decision rules; an updating entity which is adapted upon confirming for updating the individual person's verified respiratory source data set based on the received and confirmed respiratory activity data; a respiratory activity evaluation entity which is adapted for evaluating the respiratory activity data of a respiratory activity identified as an event of said individual person's respiratory activity.

Thus, a system can be provided which operates along the method as outlined above. The processing unit may be a device having a connectivity to the wearable respiratory activity recording device. The processing unit may be provided within the wearable device. The processing unit may also be provided as a base station and may be positioned beside the individual person's resting place, and which may include a charging facility for charging the wearable respiratory activity recording device. The processing unit may also be represented by e.g. a router or a Wi-Fi access point to which the wearable respiratory activity recording device is connected. The processing unit may also be realized as a repeater or a mobile communication processing unit to which the wearable respiratory activity recording device is connected via a mobile standard communication protocol, like LTE, GSM, 3G, 4G, 5G, 6G etc. The processing unit may also be realized as a server, particularly a central server for a plurality of wearable devices, where the wearable device e.g. may be connected via Wi-Fi to a router or a desktop device, and the desktop device may be connected by wire or via Wi-Fi to a remote server, where the data base is stored. It should be noted that the components of the processing unit may be distributed over different location. In particular the entities may be located remote from the data sets, in particular if the system is realized on a distributed server system.

The gist of the invention is to provide a method and a system being capable of identifying a patient's (or individual person's) respiratory, in particular cough activity among other patient's (or individual person's) respiratory, in particular cough activities and to teach a system and data base, respectively, for a more reliable identification of a patient (or individual person) and its corresponding respiratory, in particular cough activity. The respiratory/cough activity of a particular patient (or individual person) may be identified and determined by using a so-called respiratory, in particular cough fingerprint of a patient (or individual person) allowing identifying a respiratory, in particular cough activity of that patient (or individual person).

It should be noted that the above-described embodiments may also be combined and in a combined form provide a synergetic technical effect and synergetic benefits which go beyond the sum of the single technical effects and benefits.

### Brief Description of the Figures

The invention will be described by way of the following drawings:
- Figure 1: illustrates a general principle of a process of respiratory, in particular cough source identification and continuous verified respiratory, in particular cough sources accumulation according to an embodiment of the invention;
- Figure 2: illustrates a process of verified respiratory, in particular cough sources accumulation and verification according to an exemplary embodiment of the invention;
- Figure 3: illustrates an onboarding and production phase of the process of verified respiratory, in particular cough sources accumulation and verification according to an exemplary embodiment of the invention;
- Figure 4: illustrates a general process build-up of a method according to an exemplary embodiment of the invention;
- Figure 5: illustrates a principle of developing and growing of a verified respiratory activity data set according to an exemplary embodiment of the invention;
- Figure 6: illustrates an identification and allocation of a respiratory activity to a person according to an exemplary embodiment of the invention;
- Figure 7: illustrates a principle of determining an audio score according to an exemplary embodiment of the invention;
- Figure 8: illustrates a principle of determining an acceleration score according to an exemplary embodiment of the invention;
- Figure 9: illustrates a setup of a system with a processing unit implemented in a wearable unit according to an exemplary embodiment of the invention;
- Figure 10: illustrates a setup of a system with a processing unit implemented in a base station remote from a wearable unit according to an exemplary embodiment of the invention;
- Figure 11: illustrates a setup of a system with a processing unit implemented in a server or cloud remote from a wearable unit according to an exemplary embodiment of the invention; and
- Figure 12: illustrates a so-called triplet learning of an embedding model according to an exemplary embodiment of the invention.

It should be noted that same or similar reference numerals illustrate same or similar components. Along these figures exemplary embodiments of the invention will be described as follows.

### Detailed Description of Exemplary Embodiments

The invention will be described along exemplary embodiments, which are illustrated in the above referenced figures and will be described in detail in the following.

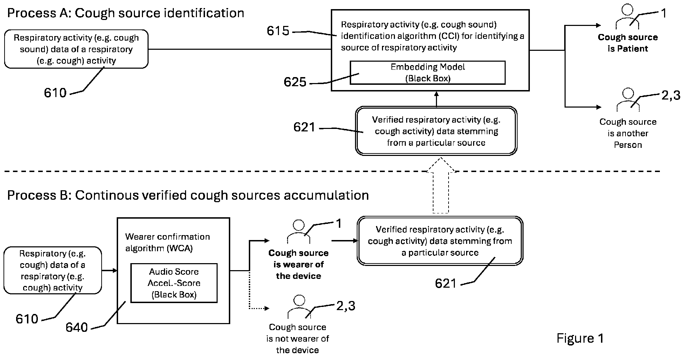

Figure 1 illustrates a general principle of a process of respiratory activity, in particular cough source identification and continuous verified respiratory activity, in particular cough sources accumulation according to an embodiment of the invention. The process as described herein has two phases.

The first phase (Process B) is an accumulation of verified respiratory activities, here cough activities. Respiratory activity data 610 are collected and verified. A verified respiratory activity data set 621 is created based on respiratory activities 610 which are for certain allocated to an individual person 1 for which respiratory activities are to be evaluated. The verified respiratory activity data set 621 serves as a basis for a later identification of respiratory activity data to be evaluated. For creating the verified respiratory activity data set 621, the received respiratory activity data 610 may be fed to a wearer confirmation algorithm WCA 640, which algorithm may use an acceleration score and/or an audio score and further parameters for creating the verified respiratory activity data set 621 for an individual person or patient 1. It should be noted that the wearer confirmation algorithm may also verify a respiratory activity data set for a further predetermined person 2 or a plurality of further predetermined persons 2, e.g., somebody who is usually in the same environment as the individual person or patient 1. This may allow a parallel procedure for a plurality of e.g. patients or may use a certain identification allocated to a further predetermined person as an indicative that this respiratory activity does not come from the patient itself. This process branch serves for providing verified respiratory activity data sets. The verified respiratory activity data sets may be further developed and enlarged during the entire process.

The second phase (Process A) serves for an identification of recorded respiratory activity data 610 based on the verified respiratory activity data set 621. The identification is carried out based on a respiratory activity identification algorithm for identifying a source of respiratory activity. This algorithm identifies the recorded respiratory activity and allocated them to a particular person, e.g. an individual person or patient 1. The identification and allocation can be carried out based on an embedding model 625, which is here considered as a black box, but will be described later in detail. The respiratory activity identification algorithm may allocate the recorded respiratory activity data to a patient 1 or to another person 2, 3. The identification or allocation may be done on a particular certainty level, which certainty level for an identification may be lower than a certainty level for confirming a respiratory activity for updating the verified respiratory activity data. In other words, if the certainty is higher than an identification level, the data will be used for allocating and a later evaluation. If the certainty lever is not only higher than an identification level, but also higher than a confirmation level, the recorded respiratory activity data can also be used for updating the verified respiratory activity data set.

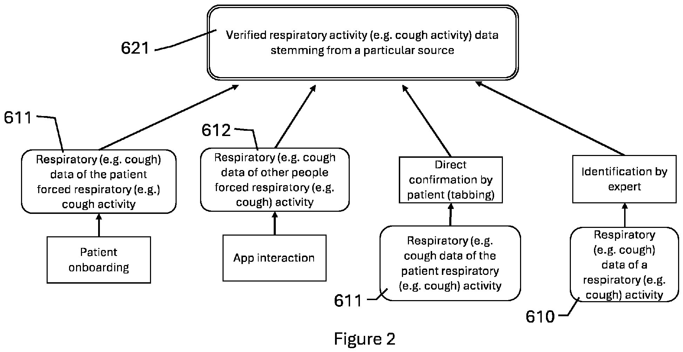

Figure 2 illustrates a process of verified cough sources accumulation and verification according to an exemplary embodiment of the invention. The verification of respiratory activity data can be carried out by different measures. To obtain a verified respiratory activity data set 621 the verification can be achieved by respiratory activity data 611 resulting from a forced respiratory activity of an individual person/patient. A patient can simulate e.g. a cough, and the characteristic of that cough(s) can be labeled as stemming from that patient 1. This also applies for other respiratory activities. This process can be carried out during a so-called onboarding phase where a base set of verified respiratory activity data set is created upon initializing the inventive method and system. Likewise, this procedure can be carried out also for a further person 2 beside the patient. Also the further person 2 can simulate a respiratory activity and the characteristic thereof can be labeled as stemming from that further person 2. Also this process can be carried out during a so called onboarding phase where a base set of verified respiratory activity data set is created upon initializing the inventive method and system. Alternatively, the onboarding of the further person 2 can be carried out later than the onboarding of the patient 1. The above-described process for the patient as well as the further person can be carried out by using e.g. a smart phone application where the respective person, i.e. the patient 1 or the further person 2 may confirm a respiratory activity, so that this respiratory activity can be directly allocated to the respective person. It is also possible that a patient confirms its respiratory activity 611 directly on the system, e.g. on a wearable unit which serves to record the respiratory activity. The patient wearing this recording device may directly confirm a respiratory activity by tabbing. As a further alternative, respiratory activity data 610 stemming from a patient and/or from a further person may be identified by an expert. The expert may analyze the respiratory activity data and may allocate the respiratory activity to either a patient or a further person or to a vague origin, other persons different from patient 1 and further/predetermined person 2. All of the described options may be used either alone or in any combination to generate a verified respiratory activity data set for a patient 1 and/or a further person 2.

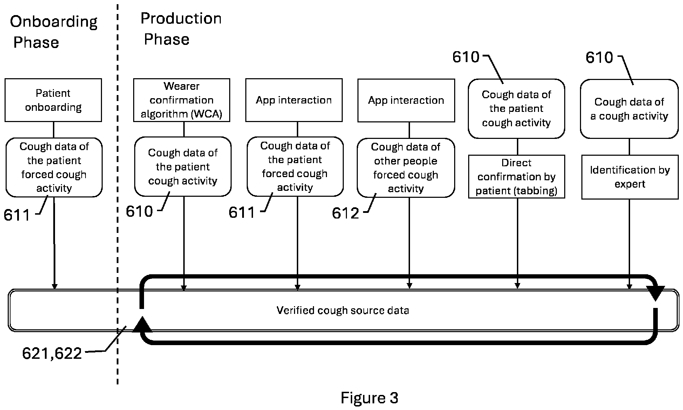

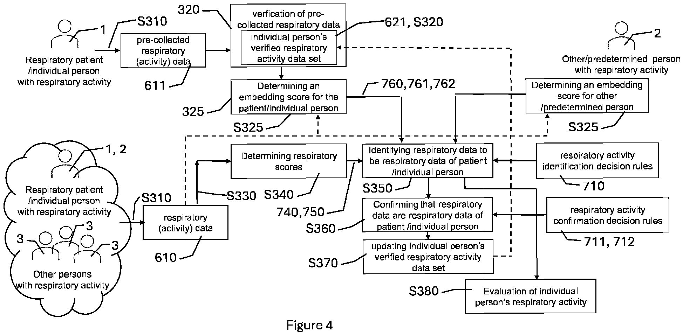

Figure 3 illustrates an onboarding and production phase of the process of verified cough sources accumulation and verification according to an exemplary embodiment of the invention. The verified respiratory activity data set usually is initiated during a so-called onboarding phase where the first base of verified respiratory activity data set is created. This can be done by collecting forced or artificially triggered respiratory activities of a patient, like coughs, which can be certainly allocated to the patient. These respiratory activity data do not need to be identified, as their origin is clear, and these respiratory activity data also do not have to be confirmed, as the certainty of origin should be 100%. In a further production phase the verified respiratory activity data set 621, 622 created during onboarding can be further updated by one or more of the following options: Confirming a respiratory activity by a wearer confirmation algorithm WCA (WCA may confirm respiratory activity data 611 of a patient or respiratory activity data 612 of a further person); Confirming respiratory activity data 611 by a user or patient 1 by using a smart phone application; Confirming respiratory activity data 612 by a further person 2, 3 by using a smart phone application; Confirming respiratory activity data by e.g. tabbing by a patient or user 1 and/or further person 2, 3; Identifying and confirming respiratory activity data 610 by an expert (expert may identify and confirm a respiratory activity of a patient or a further person); and also based on an automated process which is later described with respect to Fig. 4 substantially in steps S350, S360 and S370. Based on one or more of these measures the verified respiratory activity data set can be updated. This update can be carried out on a regular basis (e.g. every month) or upon occation (e.g. if an expert is available) or upon request (e.g. if the system detects that the data basis is not sufficient anymore or has deteriorated).

Figure 4 illustrates a general process build-up of a method according to an exemplary embodiment of the invention. A respiratory patient, also denoted as individual person 1 may have a respiratory activity which may be recorded S310 by a wearable device 200, and thus may generate respiratory activity data 611. Although not illustrated here, also a further person with respiratory activity, also denoted as predetermined or further person 2 may have a respiratory activity which may be recorded by a wearable device 200 (either worn by this person 2 or worn by the patient 1), and thus may generate respiratory activity data 612.

The pre-collected respiratory activity data 611, 612 are provided to a verification unit 320 for verification S320 of the pre-collected respiratory activity data 611, 612 so as to receive a verified respiratory activity data set 621, 622, which is then provided S325 to an embedding score determination entity 325 for determining S325 a respective embedding score 760, 761, 762 for the patient and the further person or persons 2 which is then provided to the identification entity 350. The aforementioned aspects are part of the first phase where the base is build-up based on which the later identification S350 and evaluation S380 is carried out and/or the further update with identification S350, confirmation S360 and updating S370 of the verified data 621, 622 is carried out.

For an ongoing evaluation a bulk of respiratory activity data 610 may be recorded S310 which may include respiratory activity data 611 of a patient 1 and/or respiratory activity data 612 of a further person 2 and also respiratory activity data of other persons 3 in the environment for which no verified respiratory activity data sets are available, because they are only present by incident. The respiratory activity data are provided S330 for determining S340 respiratory scores 730. Respiratory scores 730 may include an audio score 740, an acceleration score 750 and/or an embedding score 760, the latter may come from an embedding score determination unit 325, which scores will be described later in detail. After determination S325, S340 of the respiratory scores 730 the respiratory activity data are provided to an identification entity 350, for identifying S350 the origin of the respiratory activity data, which is the source of the respiratory activity, e.g. the patient 1, a further person 2 or other persons 3. The identification S350 is carried out based on the received respiratory activity data 610, the determined respiratory scores 730, 740, 750, 760 and respiratory activity identification decision rules 710. The respiratory activity identification decision rules may be general rules 710 or may be decision rules which are tailored to an individual person or patient 1 or may be decision rules which are tailored to a predetermined further person 2. If it is determined with a first particular certainty that a respiratory activity stems from a patient 1, the respective respiratory activity data may be provided to an evaluation entity 380 for further evaluation S380. If it is determined with a second particular certainty, which is higher than the first particular certainty, that a respiratory activity stems from a patient 1, the respective respiratory activity data may be provided to a confirmation entity 360 which confirms S360 the origin of the respiratory activity and based thereon updates S370 the verified respiratory activity data sets of that patient 1 in an updating entity 370. Confirmation S360 is carried out based on respiratory activity confirmation decision rules 711, 712, which may be individualized for a particular person, be it a patient 1 or a further/predetermined person 2. It should be understood that this confirmation process can be carried out for a patient 1 as well as for a further person 2, and that this can also be carried out in parallel. It should further be understood that the recorded respiratory activity data used for identification S350 and evaluation S380 at the same time can be used for identification S350, confirmation S360 and updating S370 as described above. It should also be understood that although not illustrated, the updating base as described with respect to Figure 2 and Figure 3 can also be applied to the scheme illustrated in Figure 4, wherein the aspects described in Figure 2 and Figure 3 are implemented in particular in steps S350 identification and S360 confirmation.

In the following a simplified and low-dimensional decision table is illustrated, which gives an impression on the general principle of the application of the invention, where the decision ruled are designed to come to a decision based on the different scores, which here are the audio score, the acceleration score and the embedding score:

| **Audio Score** | **Acceleration Score** | **Embedding Score** | **Decision** |
|---|---|---|---|
| High | High | High | Verified Cough |
| High | High | Low | Verified Cough |
| Low | Low | Low | Own Cough |
| Medium | Medium | Medium | Own Cough |
| Low | Low | High | Foreign Cough |
| Medium | Low | High | Foreign Cough |
| ... | ... | ... | |

The decision rules 710, 711, 712 may come to the decision that the respiratory activity data are allocated to an own cough (e.g. a patient's 1 cough or a cough of a predetermined further person 2), a foreign cough which can be identified as not belonging to the patient 1 or a further predetermined person 2, and therefore likely origins from a foreign or third person 3. It should be understood that decision tables used in practice may be of a higher dimension considering mutual correlations and may also consider dissolved stages between high and low.

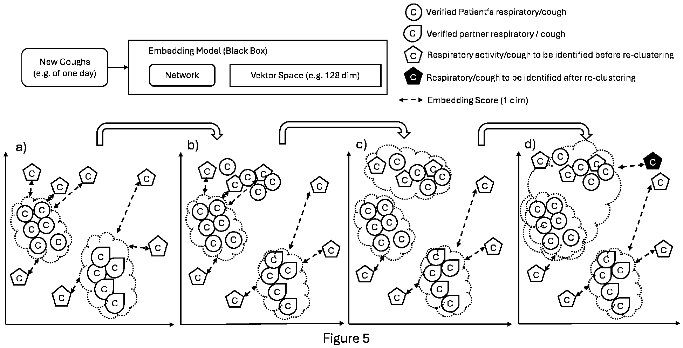

Figure 5 illustrates a principle of developing and growing of a verified respiratory activity data set according to an exemplary embodiment of the invention. New coughs, e.g. the respiratory activity data of one day, in particular the recent day, are used for feeding the embedding model. The embedding model may be based on a machine learning and machine learned model. The learned model may be used for identification S350 and evaluation S380, whereas the learning model uses identification S350, confirmation S360 and updating S370 for the learning procedure. The embedding model may map in a vector space recorded respiratory activity data to the verified respiratory activity data set. The embedding score 760 may be considered as a one-dimensional scalar value which represents the distance between recorded respiratory activity data (cough to be identified), so that in the illustrated example it can be determined or identified, whether a cough to be identified is allocated to a patient 1 or not.

If the embedding score, which is the distance between the recorded respiratory activity data and the cluster of verified respiratory activity data of a patient 1 is low, then the likelihood is high that that the recoded data origin from that patient 1. Likewise, the allocation can be carried out for a further predetermined person 2. If the embedding score, i.e. the distance, of recorded respiratory activity data to a cloud of a further predetermined person 2 is significantly lower than the embedding score, i.e. distance, to a patient 1, the recorded respiratory activity will be allocated to the predetermined person. In this case a low embedding score expresses a low distance to a respective cluster and thus a high likelihood that the respiratory activity is allocated to said cluster. This is illustrated in graph a) in Figure 5. However, also respiratory activity data which have a larger distance to the patient 1, the allocation can be overruled by one or more of the procedures described with respect to Figure 2 and Figure 3. It should be noted that the definition of the embedding score may also be inversed, in this case a high embedding score expresses a low distance to a respective cluster and thus a high likelihood that the respiratory activity is allocated to said cluster.

A number of coughs to be identified after identification may accumulate, as illustrated in graph b) of Figure 5. This accumulation may form another cluster, as illustrated in graph c) of Figure 5. The new cluster may grow together with the earlier cluster, as can be seen from graph d) of Figure 5. As can be seen from graphs a) to d), the allocation of already allocated respiratory activities (coughs to be identified before re-clustering) remains the same and in this embodiment is not updated after re-clustering. New respiratory activities (coughs to be identified after re-clustering) however follow the new cluster, as can be seen from graph d) in Figure 5. This means that the cluster, which may be considered as the verified respiratory activity data set, may vary with the time, which may be considered as the updating process of the verified respiratory activity data set. The embedding score, which may be considered as a distance between the recorded respiratory activity data to the cluster, i.e. the verified respiratory activity data set, may be determined with respect to the distance to the verified respiratory activity data set of the patient (in Figure 5 the cluster of verified patient's cough) and the verified respiratory activity data set of a further predetermined person (in Figure 5 the cluster of verified partner cough). The decision rules may allocate according to the lowest embedding score from the patient's embedding score and the partner's embedding score but may also consider other parameters. It should be noted that the illustration in Figure 5 for illustrative purposes is only a 2-dimensional vector space with a scalar distance of the recorded respiratory data. The actual embedding model may have an n-dimensional vector space depending on the complexity of the correlations and the number of relevant parameters.

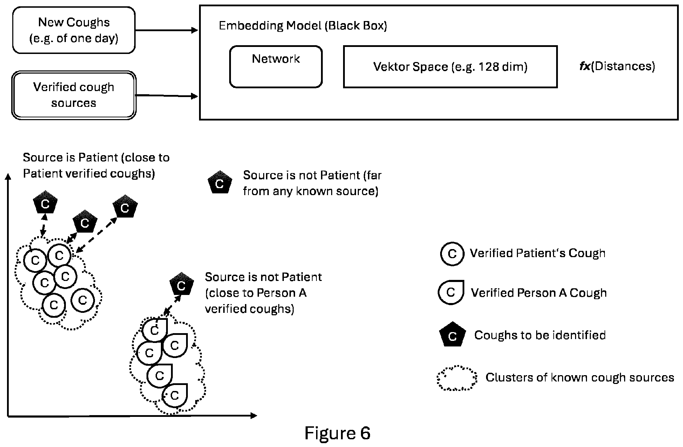

Figure 6 illustrates an identification and allocation of a respiratory activity to a person according to an exemplary embodiment of the invention. The graph illustrates that respiratory activities, here coughs to be identified, are allocated to the cluster, and thus to the verified respiratory activity data set of a patient 1 or to the verified respiratory activity data set of a further predetermined person, here the partner or person A. If an embedding score, here the distance between the cough to be identified to the respective cluster, of the cough to be identified with respect to a patient is smaller than an embedding score of the cough to be identified with respect to a partner or person A, then the cough to be detected will be allocated to the patient. If however, an embedding score of the cough to be identified with respect to a partner, here person A, is smaller than a embedding score of the cough to be identified with respect to the patient, then the cough to be detected will be allocated to the partner or person A. If a patient-related embedding score and a person A-related embedding score are large, or if the amount thereof is similar, then the cough to be detected will be allocated to neither the patient nor person A and therefore remains unconsidered. The decision logic may be implemented in the decision rules and may be more complex than illustrated in Figure 6, in particular if the decision space and the corresponding vector space has a larger number of dimensions.

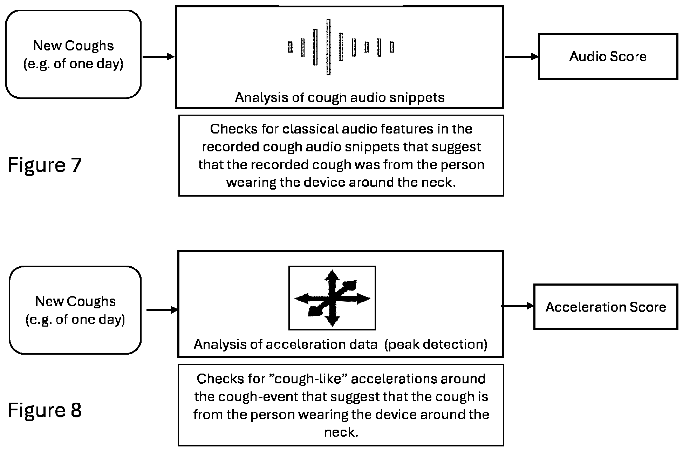

Figure 7 illustrates a principle of determining an audio score according to an exemplary embodiment of the invention. For determination of an audio score respiratory activity data, here new coughs of e.g. one day, are fed to an analyzer for analysis of cough audio snippets. The analyzer will check for classical audio features in the recorded cough audio snippets that suggest that the recorded cough or generally the respiratory activity was from the person wearing the device e.g. around the neck. The result thereof may be interpreted as an audio score. The score determination may also be carried out by determining a distance in a vector space, but also may be a simple amplitude evaluation, or a phase shift and distortion evaluation, which is higher when the physical distance from a person to the recording device is higher.

Figure 8 illustrates a principle of determining an acceleration score according to an exemplary embodiment of the invention. For determination of an acceleration score respiratory activity data, here new coughs of e.g. one day, are fed to an analyzer for analysis of acceleration data. The analyzer will check for e.g. "cough-like" accelerations around the cough-event that suggest that the cough is from the person wearing the device around the neck. The result thereof may be interpreted as an acceleration score. The score determination may also be carried out by determining a distance in a vector space, but also may be a simple amplitude evaluation, or a phase shift and distortion evaluation, which is higher when the physical distance from a person to the recording device is higher.

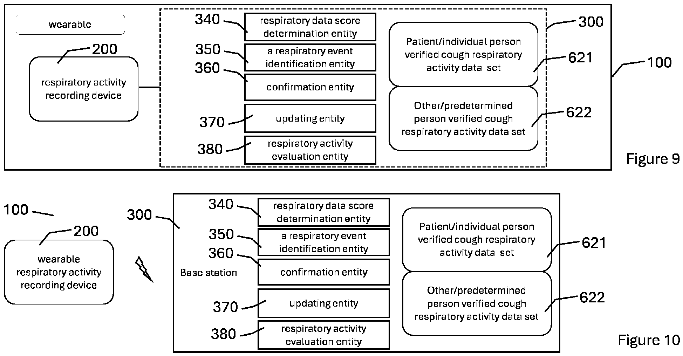

Figure 9 illustrates a setup of a system with a processing unit implemented in a wearable unit according to an exemplary embodiment of the invention. The system for identifying an individual person's respiratory activity is designed as a wearable device 100 having a recording device 200, here denoted as a respiratory activity recording device, and a processing unit 300. The processing unit 300 includes a respiratory data score determination entity 340, a respiratory event identification entity 350, a confirmation entity 360, an updating entity 370 and a respiratory activity evaluation entity 380. The functional connection is as it is described with respect to Figure 4. It should be noted that the above-described processing unit 300 is capable of carrying out both phases described with respect to Figure 1. If it is intended to use the wearable device only for evaluation purposes, the confirmation entity 360 and the updating entity 370 may be omitted. In this case the wearable device can only be used for a respiratory activity data evaluation, but not for updating the verified data. Likewise, if it is intended to use the wearable device only for updating the verified data 620, 621, 622, the evaluation entity 380 may be omitted. In this case the wearable device can only be used for updating the verified data, but not for ongoing evaluation.

Figure 10 illustrates a setup of a system with a processing unit implemented in a base station remote from a wearable unit according to an exemplary embodiment of the invention. The functional components are similar to those described with respect to Figure 9. However, the embodiment illustrated in Figure 10 has implemented in the wearable device only the recording device, whereas the processing entities are implemented in a remote base station, which may be positioned beside the patient's bed while the patient sleeps with the wearable recording device around e.g. the neck or the wrist. The wearable recording device may be controlled by a not illustrated controlling unit which may be located in the wearable device or the base station. The processing entities 340, 350, 360, 370 and 380 may be positioned in the base station 300 as the processing device, as illustrated in Figure 10. However, single components of the respiratory data score determination entity 340, the respiratory event identification entity 350, the confirmation entity 360, the updating entity 370 and the respiratory activity evaluation entity 380 may be selectively positioned in the wearable device, whereas other components of the data score determination entity 340, the respiratory event identification entity 350, the confirmation entity 360, the updating entity 370 and the respiratory activity evaluation entity 380 may be positioned in the base station. For example, the data score determination entity 340, the respiratory event identification entity 350 and respiratory activity evaluation entity 380 may be positioned in the wearable device 100 and may transfer wireless the result data to the base station, so that the wearable device 100 alone may be used for ongoing evaluation of the recorded respiratory activity data 610, and the base station is used for the updating proceed of the verified respiratory activity data 620, 621, 622. For identification and evaluation, the verified respiratory activity data sets may be transferred to the wearable device and may be stored therein until they are updated from the base station after updating the verified respiratory activity data in the base station after confirmation of the identified data.

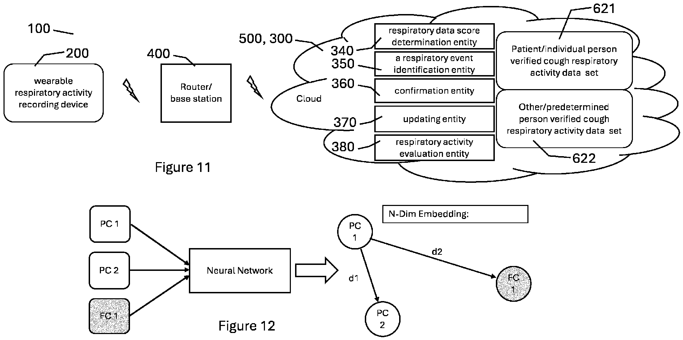

Figure 11 illustrates a setup of a system with a processing unit implemented in a server or cloud remote from a wearable unit according to an exemplary embodiment of the invention. The functional components are similar to those described with respect to Figure 9 and Figure 10. However, the embodiment illustrated in Figure 11 has implemented in the wearable device 200 only the recording device, whereas the processing entities 340, 350, 360, 370 and 380 of the processing unit 300 are implemented in a remote server or cloud 500, which may be positioned remote from the patient's bed. Such a server may be connected to a plurality of wearable devices 200 and routers 400 to which the respective wearable devices are wirelessly coupled. The wearable recording device 200 worn by a user 1 around the neck or on the wrist may be controlled by a not illustrated controlling unit which may be located in the wearable device or the remote server or cloud 500 or within the router or base station 400 beside the patient's bed. The processing entities 340, 350, 360, 370 and 380 may be positioned in the server or cloud 300 as the processing device, as illustrated in Figure 11. However, single components of the respiratory data score determination entity 340, the respiratory event identification entity 350, the confirmation entity 360, the updating entity 370 and the respiratory activity evaluation entity 380 may be selectively positioned in the wearable device, whereas other components of the data score determination entity 340, the respiratory event identification entity 350, the confirmation entity 360, the updating entity 370 and the respiratory activity evaluation entity 380 may be positioned in the router 400 or a base station (which may serve as a router 400). For example, the data score determination entity 340, the respiratory event identification entity 350 and respiratory activity evaluation entity 380 may be positioned in the base station and may transfer wireless the result data to the server or cloud, so that the wearable device 200 together with the router/base station 400 alone may be used for ongoing evaluation of the recorded respiratory activity data 610, and the server/cloud 500 is used for the updating process of the verified respiratory activity data 620, 621, 622. According to another example, the data score determination entity 340, the respiratory event identification entity 350 and respiratory activity evaluation entity 380 may be positioned in the wearable device 200 and may transfer wireless the result data via the base station/router to the server or cloud, so that the wearable device 200 alone may be used for ongoing evaluation of the recorded respiratory activity data 610, and the server or cloud is used for the updating process of the verified respiratory activity data 620, 621, 622. For identification and evaluation, the verified respiratory activity data sets may be transferred to the wearable device and base station, respectively, and may be stored therein until they are updated from the server or cloud after updating the verified respiratory activity data in the server or cloud 500 after confirmation of the identified data.

In general, a respiratory activity recording device 200, e.g. a cough monitor may be worn around the neck during the day and may monitor audio signals and acceleration. During nighttime the respiratory activity recording device 200 may be placed on a charger on the nightstand and may monitor audio signals.

Figure 12 illustrates a so-called triplet learning of an embedding model according to an exemplary embodiment of the invention. Triplet learning is a machine learning method where a model learns to distinguish between similar and dissimilar data points by analyzing triplets consisting of an anchor, a positive (similar), and a negative (dissimilar) example. The goal is to minimize the distance between the anchor and the positive example and maximize the distance between the anchor and the negative example, thereby achieving better embeddings for classification or retrieval tasks. A first cough of a patient (PC1), and a second cough of the same patient (PC2) and a cough identified as a cough of a person different from the patient, here denoted as foreign cough (FC) are fed to a neural network. The neural network determines the distances d1 and d2 between PC1 and PC2. A Loss of d1 and d2 is based on d1 and d2 and is zero if d1 < d2 and high if d1 > d1. The neural network learns to cluster PC1 and PC2 and push FC away. The neural network does not need to be re-trained for a specific patient since it simply outputs an embedding score. It should be understood that a neural network can also be trained by more than two coughs of a patient and more than one foreign cough, in particular also by coughs of the further predetermined person, although not illustrated.

### Reference numbers

- 1: patient, individual person
- 2: other person, predetermined person
- 3: person different from patient/individual person and other/predetermined person
- 100: system for identifying an individual person's respiratory activity
- 200: wearable respiratory activity recording device
- 300: processing unit
- 320: pre-collected respiratory data verification entity
- 325: embedding score determination entity
- 340: respiratory data score determination entity
- 350: respiratory event identification entity
- 360: confirmation entity
- 370: updating entity
- 380: respiratory activity evaluation entity
- 610: respiratory activity data
- 611: (pre-collected) respiratory activity data of patient/individual person
- 612: (pre-collected) respiratory activity data of other/predetermined person
- 615: respiratory activity identification algorithm
- 621: verified respiratory activity data set of patient/individual person
- 622: verified respiratory activity data set of other/predetermined person
- 625: embedding model
- 631: respiratory activity data set of patient/individual person for evaluation
- 632: respiratory activity data set of other/predetermined person for evaluation
- 640: wearer confirmation algorithm
- 710: respiratory activity identification decision rules
- 711: patient/individual person confirmation decision rules
- 712: other/predetermined person confirmation decision rules
- 730: respiration data score
- 740: audio score
- 741: audio score of patient/individual person
- 742: audio score of other/predetermined person
- 750: acceleration score
- 751: acceleration score of patient/individual person
- 752: acceleration score of other/predetermined person
- 760: embedding score
- 761: embedding score of patient/individual person
- 762: embedding score of other/predetermined person
- S310: recording respiratory activity data of a respiratory activity of at least one person
- S320: providing an individual person's verified respiratory activity data set
- S330: receiving respiratory activity data of a respiratory activity of at least one person
- S340: determining a set of respiratory data scores;
- S345: determining an embedding score
- S350: identifying received respiratory activity data of a respiratory activity
- S360: confirming whether the received respiratory activity data origin from the individual person
- S370: updating the individual person's verified respiratory activity data set
- S380: providing respiratory activity data of a respiratory activity for further evaluation

## Claims

1. A method for identifying an individual person's respiratory activity among a group of persons' respiratory activities, the method comprises iteratively:
providing (S320) an individual person's verified respiratory activity data set (621) based on pre-collected respiratory activity data (611) of said individual person's respiratory activities;
receiving (S330) respiratory activity data (610) of a respiratory activity of at least one person out of a group of persons recorded by a respiratory activity recording device (200);
determining (S340) from the received respiratory activity data (610) a set of respiratory data scores (730);
identifying (S350) the received respiratory activity data (610) of a respiratory activity to be an event out of an event group, the event group consisting of an event of said individual person's respiratory activity and an event of a respiratory activity of a person (2, 3) different from the individual person (1), wherein identifying the received respiratory activity data (610) of a respiratory activity is based on the individual person's verified respiratory activity data set (621) and a given set of respiratory activity identification decision rules (710);
confirming (S360) whether the received respiratory activity data (610) origin from the individual person (1) based on the determined set of respiratory data scores (730) and a set of individual person confirmation decision rules (711);
upon confirming: updating the individual person's verified respiratory activity data set (631) based on the received and confirmed respiratory activity data (610);
providing (S380) the respiratory activity data (610) of a respiratory activity identified as an event of said individual person's respiratory activity for further evaluation of said individual person's respiratory activity.

2. A method according to claim 1, wherein the respiratory data scores (730) include an audio score (740), the audio score is representative for a physical distance between a person and the respiratory activity recording device.

3. A method according to any one of claims 1 and 2, wherein the respiratory data scores (730) include an acceleration score (750), wherein the acceleration score is representative for an acceleration of a person's anatomy during a respiratory activity.

4. A method according to any one of the preceding claims, wherein confirming (S360) the received respiratory activity data (610) of a respiratory activity is based on the individual person's verified respiratory activity data set (621), a given set of respiratory activity confirmation decision rules (711) and an individual person's embedding score (761), wherein the embedding score is representative for a confidence level of the identification of a respiratory activity to be an event of said individual person's respiratory activity.

5. A method according to any one of the preceding claims, wherein confirming (S360) the received respiratory activity data (610) of a respiratory activity is based on the individual person's verified respiratory activity data set (621), a given set of respiratory activity confirmation decision rules (711, 712), an individual person's embedding score (761) and a predetermined person's embedding score (762), wherein the respective embedding score (761, 762) is representative for a confidence level of the identification of a respiratory activity to be an event of said predetermined person's respiratory activity.

6. A method according to claim any one of claims 4 and 5, wherein at least one of the individual person's embedding score (761) and the predetermined person's embedding score (762), respectively, is determined based on a difference between on the one hand received respiratory activity data (610) and on the other hand individual person's verified respiratory activity data set (621) and predetermined person's verified respiratory activity data set (622), respectively.

7. A method according to any one of the preceding claims, wherein the respiratory activity identification decision rules (710) and/or the confirmation decision rules are represented by at least one of a look-up table, an algorithm and a model representing a relation between the respiratory data scores (740, 750, 760) and the events out of the event group.

8. A method according to any one of the preceding claims, further providing for at least one predetermined person (2) out of the group of persons a predetermined person's verified respiratory activity data set (622) based on pre-collected respiratory activity data (610) of said predetermined person's respiratory activities , wherein the event group further comprises the event of respiratory activity of the at least one predetermined person out of the group of persons, wherein identifying (S340) the respiratory activity data (610) of a respiratory activity to be an event of the event group, is based on a set of respiratory activity identification decision rules (710), the individual person's verified respiratory activity data set (621), the predetermined person's verified respiratory activity data set (622) and the set of respiratory data scores (730).

9. A method according to any one of the preceding claims, wherein upon failed confirming (S360) or bypassed confirming: the received respiratory activity data (610) are provided for verifying by an expert; and upon verifying: updating the individual person's verified respiratory activity data set (621) or predetermined person's verified respiratory activity data set (622) based on the received and verified respiratory activity data (610).

10. A method according to any one of the preceding claims, wherein upon failed confirming (S340) or bypassed confirming: the received respiratory activity data (610) are provided for verifying by said individual person (1); and upon verifying: updating (S370) the individual person's verified respiratory activity data set (621) based on the received and verified respiratory activity data (610).

11. A method according to any one of the preceding claims, wherein upon failed confirming (S340) or bypassed confirming: the received respiratory activity data (610) are provided for verifying by said predetermined person (2); and upon verifying: updating the predetermined person's verified respiratory activity data set (622) based on the received and verified respiratory activity data (610).

12. A method according to any one of claims 4 to 11, wherein for determination (S340) of the embedding score (760, 761) the respiratory activity data (610) of a number of respiratory activities of the individual person's verified respiratory activity data sets (621) are clustered as a verified cluster in an in an n-dimensional embedding model space, wherein the embedding score (760, 761) is determined as a distance between the received respiratory activity data (610) of a respiratory activity and the verified cluster in the embedding model space.

13. A method according to any one of the preceding claims, wherein upon receiving of respiratory activity data (610) of a respiratory activity and identification (S340) of said respiratory activity data (610) of a respiratory activity to be an event of individual person's respiratory activity, the number of respiratory activities of the individual person's verified respiratory activity data set (621) is supplemented based on the received respiratory activity data (610), and the supplemented individual person's verified respiratory activity data set (621) is re-clustered as an updated verified cluster in an in an n-dimensional embedding model space.

14. A method according to any one of claims 4 to 13, wherein determining the embedding score (760, 761) is based on the individual person's verified respiratory activity data set (621) updated based on recently received respiratory activity data (610) related to the individual person's respiratory activity.

15. A system for identifying an individual person's respiratory activity among other person's respiratory activities, the system (100) comprises:
a wearable respiratory activity recording device (200) being adapted for sensing and recording respiratory activity data (610) of a respiratory activity of at least one person out of a group of persons (1, 2, 3) including an individual person (1);
a processing unit (300) having access to an individual person verified respiratory activity data set (621) based on pre-collected respiratory activity data (611) of said individual person's respiratory activities;
wherein the processing unit (300) comprises:
a respiratory data score determination entity (340) which is adapted for determining from the received respiratory activity data (610), a set of respiratory data scores (730);
a respiratory event identification entity (350) which is adapted for identifying the received respiratory activity data (610) of a respiratory activity to be an event out of a group, the group comprising the event of said individual person's respiratory activity and the event of a respiratory activity of a person different from the individual person, wherein identifying the received respiratory activity data of a respiratory activity is based on the individual person's verified respiratory activity data set (621) and a given set of respiratory activity identification decision rules (710);
a confirmation entity (360), which is adapted for confirming whether the received respiratory activity data (610) origin from the individual person (1) based on the set of respiratory data scores and a set of individual person confirmation decision rules (711);
an updating entity (370) which is adapted upon confirming for updating the individual person's verified respiratory source data set (621) based on the received and confirmed respiratory activity data (610);
a respiratory activity evaluation entity (380) which is adapted for evaluating the respiratory activity data (610) of a respiratory activity identified as an event of said individual person's respiratory activity.
